# EUROPEAN PATENT APPLICATION

(11) **EP 4 616 883 A1**
(43) Date of publication of application: **17.09.2025**
(21) Application number: 24162952.6
(22) Date of filing: 12.03.2024
(51) Int. Cl.: A61M 5/24, A61M 5/315

(54) **DOSE AND DISPENSING MECHANISM FOR AN INJECTION DEVICE**

(71) Applicant: Ypsomed AG, 3401 Burgdorf (CH)
(72) Inventor: Kalbermatter, Gabriel, 3400 Burgdorf (CH); Glauser, Oliver, 3008 Bern (CH); Hirschel, Jürg, 3007 Bern (CH)
(74) Representative: Kleiner, Stefan

(57) **Abstract**

The invention relates to dose and dispensing mechanism comprising a housing (10), a plunger rod (60), a dosing click member (80) comprising axially extending dosing click teeth (82) adapted to engage axially extending counter dosing click teeth (72) and the mechanism further comprises a dispensing click member (90) inside the housing and comprising dispensing click teeth (91) adapted to engage counter dispensing click teeth (71). In a dose setting position the drive member (40) is operatively coupled to the dosing click member (80) to rotate it relative to the housing which causes the dosing click teeth (82) to slide over the counter dosing click teeth (72) and in a dispensing position the drive member (40) is operatively coupled to the dispensing click member (90) to rotate it relative to the housing which causes the dispensing click teeth (91) to slide over the counter dispensing click teeth (71).

## Description

### FIELD OF THE INVENTION

The present invention relates to injection devices or medicament delivery devices for injecting, delivering, administering, infusing or dispensing substances and/or liquids such as insulin or hormone preparations. It departs form a dose and dispensing mechanism for a drug delivery device for dispensing a liquid drug from a reservoir through an injection needle. The dose and dispensing mechanism comprises a housing, a plunger rod, a dosing click member comprising axially extending dosing click teeth adapted to engage axially extending counter dosing click teeth, a dispensing click member comprising dispensing click teeth adapted to engage counter dispensing click teeth and a drive member movable relative to the housing and adapted to be releasably coupled to the dosing click member or to the dispensing click member.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the injection pen. A dose is dispensed by inserting the needle into a suited portion of human skin and by moving the piston manually or by pressing a release button of an automatic injection device. Automatic injection devices may comprise an electronic dose dial mechanism to automatically set a dose.

CH 709 878 A2 discloses a reusable injection pen with a cartridge holder releasably attachable to a pen housing. A dose sleeve is rotatable relative to the housing to set a dose. To dispense the set dose a user presses a button and moves the dose sleeve together with a clutch sleeve relative to the housing in dispensing direction. This decouples a ratchet sleeve from the housing. During dispensing the dose sleeve and the ratchet sleeve are rotated and ratchet teeth slide over saw teeth of the housing thereby generating a click sound. The rotating dose sleeve rotates a drive sleeve which is in threaded connection with the plunger rod. The latter is splined to the housing and is moved axially without rotation during dispensing.

CH 715854 A2 discloses a further reusable injection pen. The dispensing mechanism includes a metal ring with axially extending click arms and rotationally coupled to the housing. During dispensing axially extending saw teeth of a rotating drive sleeve slide over the click arms and thus generate a click sound. During dispensing the rotating drive sleeve rotates the piston rod which is in a threaded connection to the housing and is thus screwed in dispensing direction.

EP 1003581 B1 discloses an injection pen comprising a dose sleeve rotatable relative to the housing to set a dose, a clutch sleeve coaxially arranged inside the dose sleeve, a drive sleeve located inside the clutch sleeve and rotatable to dispense a set dose and a plunger rod in threaded connection with the housing. During dose setting teeth of the dose sleeve slide over teeth of the clutch sleeve at the proximal end and during dispensing distally arranged and radially extending click arms slide over saw tooth of the housing to generate a click sound. The plunger rod is coupled to the housing by a thread and therefore screwed in distal dispensing direction during dose dispensing.

### DESCRIPTION OF THE INVENTION

It is an objective of the invention to reduce the user force required to dispense a dose while providing a user feedback during dose setting and dose dispensing in a space-saving design.

This objective is achieved by a manually driven and reusable injection device according to the independent claims. Preferred embodiments are evident from the dependent claims.

The invention relates to a manually driven and reusable injection device for dispensing a liquid drug from a reservoir, in particular a cartridge or syringe, through an injection needle. The dose and dispensing mechanism comprises
- housing defining longitudinal axis;
- a plunger rod arranged inside the housing and displaceable relative to the housing for dispensing the liquid drug from the reservoir;
- a dose setting member inside the housing and movable out of the housing for setting a dose, wherein an axial position of the dose setting member relative to the housing is indicative of a set dose;
- a dose click member inside the housing and comprising axially extending dosing click teeth adapted to engage axially extending counter dosing click teeth;
- a dispensing click member inside the housing and comprising dispensing click teeth adapted to engage counter dispensing click teeth;
- a drive member movable relative to the housing and adapted to be operatively and releasably coupled to the dosing click member or to the dispensing click member to transfer a drive movement to the dosing click member or to the dispensing click member, respectively. The drive member can be in a dose setting position and in a dispensing position.

During dose setting (drive member dose setting position) the drive member is coupled to the dosing click member to rotate the dosing click member relative to the housing such that the dosing click teeth slide over the counter dosing click teeth. During dispensing (drive member dose dispensing position) the drive member is decoupled from the dosing click member and operatively coupled to the dispensing click member to rotate the dispensing click member relative to the housing such that the dispensing click teeth slide over the counter dispensing click teeth. In the dispensing position the drive member is operatively coupled to the plunger rod.

The mechanism further includes a ratchet member which comprises both the counter dosing click teeth and the counter dispensing click teeth. The counter dosing click teeth are distinct and spaced apart from the counter dispensing click teeth. The ratchet member can be in first axial position in which the ratchet member engages the dispensing click member and the counter dispensing click teeth engage the dispensing click teeth and the ratchet member can be a second axial position in which the ratchet member is axially separated and spaced apart from the dispensing click member and correspondingly the counter dispensing click teeth are axially separated from the dispensing click teeth.

The plunger rod comprises a flange rotatably connected to plunger rod with a distal flange contact surface adapted to serve as a contact for user-initiated force application for proximal displacement of the plunger rod when the ratchet member is in its second axial position. The contact surface has preferably a diameter or a maximum dimension at least double of the diameter of the plunger rod. The plunger rod is operatively connected (direct or indirect via intermediate member) to the housing by a threaded connection such that the plunger rod can be screwed in dispensing direction when the drive member is in its dispensing position.

According to the invention the dispensing mechanism includes a ratchet member providing both the counter dosing click teeth for a click sound during dose setting and the counter dispensing click teeth for a click sound during dose dispensing. The counter dosing click teeth and the counter dispensing click teeth are different teeth which are distinct and separated from each other. The counter dosing click teeth and the counter dispensing click teeth being arranged both on the same component allows for a space saving design. Moreover, the ratchet member is used in combination with a plunger rod that is in threaded connection with the housing. Compared to a plunger rod that is axially movable without rotation (linearly guided) the claimed screwing plunger rod has a reduced friction. As the friction is reduced for the dispensing movement the user force required to move the plunger rod is reduced compared to a mechanism with plunger rod that is axially shifted without rotation during dispensing.

The ratchet member is movable relative to the housing along the longitudinal axis. The plunger rod is preferably operatively (direct or indirect via intermediate member) coupled to the dispensing click member. A rotation of the plunger rod is thus transferred to the dispensing click member which rotates relative to the ratchet member and the dispensing click teeth slide over and engage the counter dispensing click teeth when the ratchet member is in its first axial position. The engagement between the dispensing click teeth and the counter dispensing click teeth may allow a rotation of the plunger rod in a first direction and may prevent, limit or brake a rotation of the plunger rod in a second, counter direction. The ratchet member may be moved into its second position in which the ratchet member and thus the dispensing click teeth and counter dispensing click teeth are decoupled and spaced apart from each other and thereby, for example, bypass a reverse lock for the plunger rod provided by the dispensing click member. This allows to rotate the plunger rod in both directions. The plunger rod may, for example, be screwed back proximally to reset the device and to prepare the injection device for a new injection. The housing may include a linear guide, for example, guiding rails guiding the ratchet member along the longitudinal axis.

That means the ratchet member is axially movable relative to the housing to rotationally couple or rotationally decouple the ratchet member and the dispensing click member. This coupling or decoupling of the ratchet member may be initiated by an attachment or detachment of a reservoir holder to the housing or/and by the presence of a cartridge in the cartridge holder.

The dosing click member and the dispensing click member are two separate components movable relative to the housing and independent from each other. The dosing click member and the dispensing click member may be, for example, sleeve-shaped, ring-shaped, disc-shaped or cylindrical components within the housing.

The dosing click member and the dispensing click member each comprises teeth adapted to cooperate with counter teeth to generate an audible and tactile feedback to the user in form of clicks. The dosing click teeth and preferably also the dispensing click teeth extend axially. That means a tooth high (or dept of the tooth) of the dosing click teeth and dispensing click teeth and the corresponding counter teeth extends along the longitudinal direction. When the teeth and counter teeth slide along each other the dosing click member, the dispensing click member or/and the ratchet member having the corresponding counter teeth may travel a short distance along the longitudinal axis.

The drive member can be operatively and releasably coupled the dosing click member or/and to the dispensing click member to transfer a drive movement. The drive member is preferably coupled to a user actuated component such as a dispensing button or to an automatic drive such as an electric motor. Therefore, the drive member is rotated by a manual or automatic drive to dispense the set dose. A clutch may be provided to selectively couple the drive member to the dosing click member, to the dispensing click member or to both the dosing click member and the dispensing click member.

The drive member is operatively and rotationally coupled to the plunger rod to rotate the plunger rod and to screw to plunger rod in distal dispensing direction. The drive member may be axially movable relative to the plunger rod or it may be axially fixed.

The term "operatively coupled" in the present description means that a first member can be directly coupled to a second member or that the first member may be coupled indirectly, via one or more intermediate member, to the second member. The term operatively means that a movement is transferred from the first component to the second without the limitation that the first member has to be in direct contact with the second member.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the injection device carrying an injection needle or an injection cannula, whereas the term "proximal" is meant to refer to the opposite direction or end pointing away from the needle or cannula.

The term "injection device" or "injector" refers to a device that is removed from the injection site after each medication event or drug delivery process, whereas the term "infusion system" refers to a device with a cannula or needle that remains in the skin of the patient for a prolonged period of time, for example, several hours.

The dosing click member is preferably sleeve-shaped or ring-shaped and the dosing click teeth are arranged on a front surface of the sleeve. This provides for a space-saving design as other components of the mechanism may be arranged inside the dosing click sleeve. Alternatively, the dosing click member may be, for example, disc-shaped.

The drive member may be arranged coaxially inside the dosing click sleeve such that the drive member can rotate relative thereto. The drive member may be releasably and rotationally coupled to the click sleeve, for example, by a releasable spline engagement.

Preferably, the drive member and the dosing click member are axially movable relative to each other to rotationally couple or decouple the drive member and the dosing click member. The releasable coupling may be provided, for example, by splines, by teeth or by a protrusion and a groove engagement. Alternatively, the coupling or decoupling movement between the drive member and the dosing click member may be a rotation or a helical movement.

The dosing click member may be biased by a spring member such that the dosing click teeth are constantly pressed onto the counter dosing click teeth. That ensures that the teeth of the biased dosing click member are in contact with the counter teeth and can cooperate with the counter teeth. The spring member may be, for example, a mechanical spring, an elastomer component.

In a preferred embodiment, in the dose setting position the drive member is rotationally decoupled from the plunger rod such that the drive member cannot transfer a drive movement to the plunge rod. In the dispensing position the drive member is preferably rotationally coupled to the plunger rod and preferably also to the dispensing click member such that a drive movement of the drive member can be transferred to the plunger rod and to the dispensing click member.

The drive member is preferably movable between the dose setting position and the dispensing position by a (axial) movement along the longitudinal axis relative to the housing.

Preferably, the dosing click teeth and the dispensing click teeth of the ratchet member both arranged in a respective circle and the circles are coaxially positioned to each other. That means the dosing click teeth or the dispensing click teeth are arranged in an outer circle and the other of the dosing click teeth and the dispensing click teeth are positioned in an inner circle. Such an arrangement provides a space-saving design.

The ratchet member may be sleeve-shaped or ring-shaped and the dispensing click member may be arranged coaxially inside the ratchet member. As stated above, a design with sleeve-shaped components enables a space-saving mechanism as the dispensing click member may be arranged inside the sleeve-shaped ratchet member.

Preferably, the dosing click teeth are arranged on an annular front surface of the sleeve-shaped ratchet member and the dispensing click teeth are preferably arranged on an inner wall or surface inside the sleeve-shaped ratchet member. The wall or surface may be oriented perpendicular to the longitudinal axis. That means the dosing click teeth and the dispensing click teeth are both arranged in a plane perpendicular to the longitudinal axis but the planes may be axially spaced apart from each other.

The ratchet member, the dosing click member and the dispensing click member are preferably concentrically arranged to each other. That provides a space saving design in particular with respect to the dimension in the longitudinal direction.

The dispensing click teeth preferably extend axially in distal direction and the counter dispensing teeth may extend axially in proximal direction such that the teeth and counter teeth can slide over each other during dose dispensing. Extending axially means that a tooth high of the teeth extends in the longitudinal direction and not in a radial direction.

The dispensing click member is preferably biased by a spring member, in particular a mechanical spring, towards the ratchet member such that the dispensing click teeth are pressed onto the counter dispensing click teeth.

In a preferred embodiment the dispensing mechanism further comprises a stop member in threaded connection with the plunger rod and splined to the drive member such that the stop member is movable axially relative to the drive member but is prevented from being rotated relative to the drive member. The stop member travel along the plunger rod when a dose is set and remains in the position during dose dispensing. The stop member is thus adapted to prevent a setting of a dose that exceeds the capacity of the reservoir. Therefore, the user cannot dial a dose that exceeds the amount of remaining drug inside the reservoir.

The dose and dispensing mechanism preferably comprises further a drive nut which is rotationally coupled to plunger rod but is movable relative to the plunger rod. The drive nut may be arranged coaxially inside the drive member and the drive nut may be releasably coupled to drive member such that a rotational movement of the drive member is transferred to the drive nut. The drive member and the drive nut may be, for example, coupled or decoupled by a relative movement along the longitudinal axis. Hence, the plunger rod is driven by the drive member via drive nut which may act as a clutch.

The drive nut may be a sleeve or a ring surrounding the plunger rod and coupled to the plunger rod by a spline engagement such that a rotation of the drive nut is transferred to the plunger rod but an axial movement of the drive nut relative to the plunger rod is allowed.

The drive nut is preferably axially coupled to the housing (direct or indirect via intermediate member) but is allowed to rotate relative thereto.

The drive member may comprise a sleeve-shaped coupling portion including a protrusion or groove (preferably on an inner surface) of the sleeve and the drive nut may include the other of the protrusion and groove (preferably on an outer surface) to rotationally couple the drive nut with the drive member. The spline connection provides a quick and easy coupling or decoupling between the drive member and the drive nut.

The drive member may be axially shifted relative to the housing to couple or uncouple the drive member with the drive nut. For example, during dose setting the drive member is not coupled to the drive nut and during dose dispensing the drive member may be coupled to the drive nut to transfer a rotational movement. Preferably, in the dose dispensing position of the drive member the drive member is coupled to the drive nut and in the dose dispensing position of the drive member it is not coupled to the drive nut.

The dispensing click member is preferably rotationally coupled to the plunger rod by the drive nut. That means a rotation of the drive nut (driven by the drive member) may be directly transferred to the dispensing click member to generate the dispensing click for the user during dose dispensing.

In a preferred embodiment the dose and dispensing mechanism further comprises a clutch member axially movable relative to the housing and rotationally coupled and immovable relative to the housing (for example by spline engagement) and in threaded connection with a dose member. In a first axial position of the clutch member (during dose setting) the drive member is rotationally decoupled such that a rotation of the drive member is not transferred to the plunger rod. In a second axial position which is different from the first axial position of the clutch member the drive member is coupled (preferably indirect via intermediate member) to the plunger rod and preferably additionally to the dispensing click member such that a rotation of the drive member is transferred to the plunger rod.

The clutch member is preferably axially coupled to the drive member but can be rotated relative to the drive member.

To start the injection the user may press a button and thereby move the clutch member from its first axial position into its second axial position in which the dose member and the drive member are coupled to the plunger rod to transfer a rotational movement of the drive member to the plunger rod. The clutch member may be sleeve-shaped and may comprise an inner thread and the dose member may comprise an outer thread engaging the inner thread.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a sectional view of an injection pen with a dose and dispensing mechanism according to the invention, wherein the cut for the view runs along the longitudinal axis;
- Fig. 2: depicts an enlarged view of a middle section of the injection pen of figure 1;
- Fig. 3: depicts the injection pen after a dose is set;
- Fig. 4: depicts the injection pen when the dispensing button is depressed;
- Fig. 5: depicts a perspective view of the ratchet sleeve of the dose and dispensing mechanism;
- Fig. 6: depicts a perspective view of the dispensing click disc;
- Fig. 7: depicts a side view of the plunger rod with the stop nut;
- Fig. 8: depicts an enlarged view of the dose and dispensing mechanism in a reset configuration;
- Fig. 9: depicts a proximal portion of a second embodiment of the injection pen according to the invention and
- Fig. 10: depicts a sectional view of the proximal portion of figure 9.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the present description the term "distal" refers to the side where the injection needle is located. This is on the left-hand side in the figures. The term "proximal" refers to the opposite side or rear end of the device and is on the right-hand side in the figures.

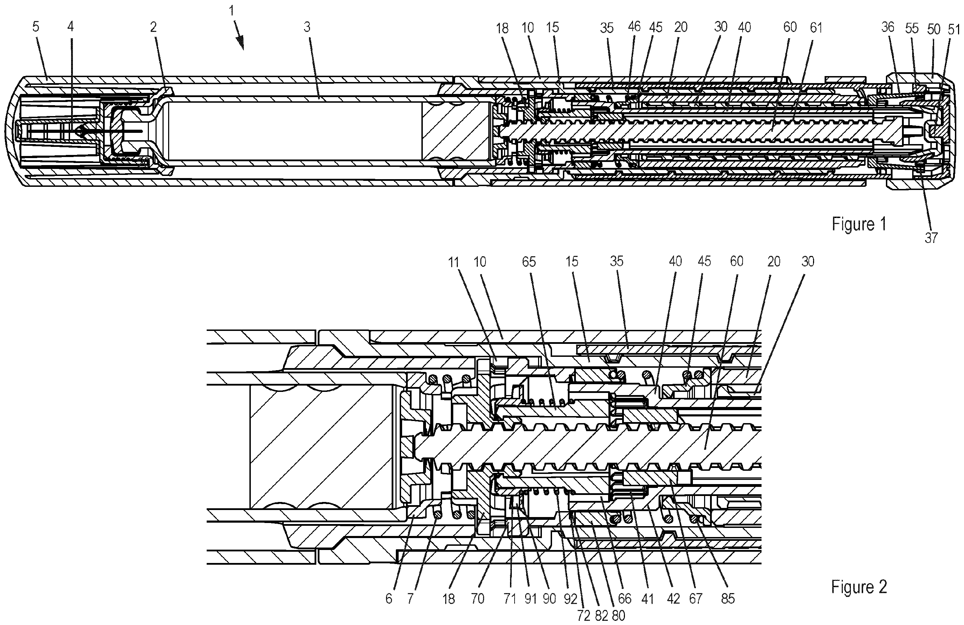

Figure 1 depicts injection device in form of a reusable injection pen 1 with a dose and dispensing mechanism according to the invention. The injection pen 1 includes a housing 10 accommodating the dose and dispensing mechanism, a cartridge holder 2, a cartridge 3 with the liquid drug and a pen cap 5 surrounding the cartridge holder 2 and an injection needle 4 mounted on a distal end. The cartridge holder 2 can be attached and detached from a housing insert to replace a cartridge 3 as known in the art.

In the following paragraphs the components of the dose and dispensing mechanism according to the invention are described. Subsequently, the function and in particular the dose setting and dispensing operation is described.

Figure 1 depicts a sectional view where the cut for the section runs along the longitudinal axis of the injection pen. As shown, the housing 10 is the outermost part and an insert sleeve 15 is fixedly connected to the housing 10 and comprises on its outside a thread engaging an inner thread of a scale drum or number sleeve 35 which is located coaxially outside the insert sleeve 15 and inside the housing. The scale drum 35 indicates the dose values to the user.

Coaxially inside the housing insert sleeve 15 a clutch sleeve 20 is located and splined to an inner surface of the insert sleeve 15 such that the clutch sleeve 20 is axially movable but prevented from being rotated relative to the insert sleeve 15 and the housing 10. The clutch sleeve 20 includes a thread segment on its inner surface engaging an outer thread of a dose sleeve 30 which is coaxially inside the clutch sleeve 20. On a proximal end the dose sleeve 30 is fixedly connected to a collar 36 which comprises cams 37 on its outside adapted to be releasably coupled to grooves 55 inside a dose knob 50 (figure 1). The dose knob 50 can thus be rotationally and releasably coupled to the dose sleeve 30 via cams 37 and grooves 55. Due to its thin-walled shape the collar 36 can be deformed if a certain force threshold is exceeded. That means, for example, if the dose sleeve 30 with the collar 36 is rotationally blocked and if the user continues to apply a torque to the rotationally coupled dose knob the collar 36 may deform (e.g. deflect radially inwards) and the cams 37 may move relative to the grooves 55. The collar 36 thus provides an overload clutch that may preserve the mechanism from damage if the torque exceeds a threshold. The dose knob 50 further includes a cam-shaped bearing 51 providing a support for the dose knob 50 such that the latter can be rotated relative to the dose sleeve 30 in a decoupled state.

The dose sleeve 30 is further rotationally connected to the scale drum 35 via spline engagement but is movable axially relative to the scale drum.

Inside the dose sleeve 30 a drive sleeve 40 is coaxially positioned and splined to the dose sleeve 30 such that a relative rotation is prevented but a relative axial movement between the drive sleeve 40 and the dose sleeve 30 is allowed. The drive sleeve 40 is axially connected to the clutch sleeve 20 by a connecting disc 45 such that an axial movement of the clutch sleeve 20 is transferred to the drive sleeve 40 but a relative rotation is not transferred. The drive sleeve 40 is coupled to a plunger rod 60 via a drive nut. The plunger rod 60 with an outer thread 61 is arranged inside the drive sleeve 40 and the plunger rod 60 is in threaded connection with a bearing disc 18, which is fixedly connected to the housing 10. A distal end of the plunger rod is rotatably connected to a flange 64 (figure 2) in turn abutting a proximal face of a piston inside the cartridge 3. Furthermore, the flange provides for a non-rotating contact surface for manual reset or proximal retraction movement of the plunger rod upon cartridge replacement.

Figure 2 depicts an enlarged view of a middle portion of the sectional view of the injection pen 1. As shown in figure 2 the drive sleeve 40 includes in a distal portion a coupling part 42 including splines 41 on an inner surface adapted to engage corresponding splines or grooves 66 on an outside of the sleeve-shaped drive nut 65 surrounding the plunger rod 60. The drive nut 65 is axially coupled to the bearing disc 18 and thus to the housing but is allowed to rotate relative thereto. Moreover, the drive nut 65 is splined to the plunger rod 60 and hence rotationally coupled to it.

The clutch sleeve 20 and drive sleeve 40 are axially movable relative to the housing. That is, during dose setting the drive sleeve 40 can be in a proximal dose setting position in which the spline engagement to the drive nut 65 is released allowing a rotation of the drive sleeve 40 relative to the drive nut 65 and thus a rotation of the drive sleeve 40 is not transferred to the plunger rod 60. The drive sleeve 40 can be further in a distal dispensing position in which the spline engagement is established and a rotation of the drive sleeve 40 is transferred to the plunger rod 60 via drive nut 65.

Radially outside of the coupling part 42 and releasably and rotationally coupled to the drive sleeve a dosing click ring 80 is located comprising on a distal front surface axially extending dosing click teeth 82 engaging axially extending counter click teeth 72. The latter are arranged on a proximal front surface of a ratchet sleeve 70. The dosing click ring 80 is biased in distal direction by a reset spring 85 which abuts on a distal side onto the dosing click ring 80 and on a proximal side on the connecting disc 45. Therefore, the dosing click teeth 82 are pressed onto the counter click teeth 72. The drive sleeve 40 comprises in a distal portion radially extending protrusions adapted to be engaged in corresponding grooves in the dosing click ring 80 to rotationally couple the drive sleeve 40 to the dosing click ring 80.

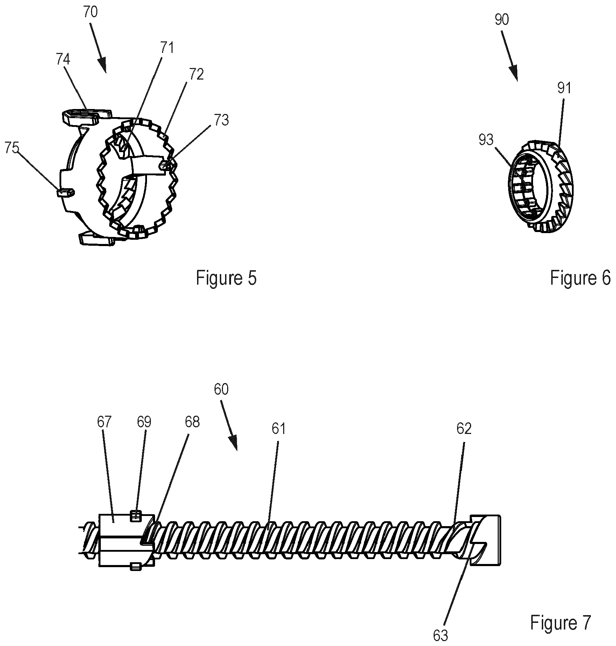

The ratchet sleeve 70 is located proximally to the bearing disc 18 and is rotationally fixed to the housing 10 but axially moveable relative thereto for a device reset operation. Figure 5 depicts a perspective view of the ratchet sleeve 70. As shown in figure 5 the counter dosing click teeth 72 are arranged on an outer circle on a proximally oriented front face of the ratchet sleeve 70. On an inner annular surace or wall perpendicular to the longitudinal axis are circularly arranged counter dispensing click teeth 71 and likewise oriented axially and adapted to engage dispensing click teeth 91 of a dispensing click disc 90. On its outside the ratchet sleeve 70 comprises two oppositely arranged guiding rails 74 which are connected to each other and thus forming a U-shape. The rails 74 rotationally couple the ratchet sleeve 70 to the bearing disc 18. Moreover, a distal end of the U-shaped rails 74 provides a contact surface for the cartridge holder. In an attached position a proximal end of the cartridge holder presses on a distal end of the rails 74 thereby holding the ratchet sleeve in place as the ratchet sleeve is biased in distal direction by the spring 85. The ratchet sleeve 70 further comprises two oppositely arranged and axially extending cams 75 which rotationally couple the ratchet sleeve 70 to a linear guide 11 (figure 1) of the housing 10. On its inside the ratchet sleeve 70 further includes four radially inward extending protrusions 73 which act as guiding means to guide the drive sleeve 40 to ensure that the protrusions and grooves correctly engage each other when the drive sleeve 40 is about to couple with the dosing click ring 80 during proximal retraction of the drive sleeve.

Figure 6 depicts a perspective view of the dispensing click disc 90. It has on its distal side the dispensing click teeth 91 and which are biased onto the counter dispensing click teeth of the ratchet sleeve 70 by a mechanical spring 92 which is located between the dispensing click disc 90 and a ledge of the drive nut 65, see figure 2. The dispensing click teeth 91 and the counter dispensing click teeth 71 are saw teeth adapted to slide along each other in a first direction but preventing a relative rotation in the counter second direction. On its inner surface the dispensing click disc 90 comprises the grooves 93 which accommodate corresponding protrusions of the drive nut 65 to rotationally couple the dispensing click disc 90 to the drive nut 65.

A stop nut 67 is in treaded engagement with the outer plunger rod thread 61. Figure 7 depicts a side view of the plunger rod 60 with the stop nut 67. The stop nut 67 comprises a stop surface 68 oriented in a plane parallel to the longitudinal axis and adapted to abut a counter stop surface 63 of an end portion of the plunger rod 60. The outer thread 61 is has a thread pitch that changes at the proximal end of the thread. As show in figure 7 the thread includes an end thread segment 62 that has a larger thread pitch (larger thread angle) than the rest of the plunger rod thread 61. Due to that end thread segment 62 the force acting on the plunger rod end portion can be reduced when the stop nut 67 abuts the stop surface 63.

The stop nut 67 further includes on its outside three circumferentially arranged cams 69 that are guided in corresponding grooves on an inner surface of the drive sleeve 40. The stop nut 67 can thus move axially relative to the drive sleeve 40 but is prevented from being rotated relative thereto.

As shown in figure 2 distally to the bearing disc 18 a cartridge support 6 is located which is biased in distal direction by a cartridge spring 7 pushing the support 6 against a proximal end rim of the cartridge. The biased cartridge support 6 ensures that the cartridge is biased in distal direction and thus safely held in place within the cartridge holder 2.

To set a dose the user grasps the dose knob 50 and rotates it. The rotation and the torque are transferred to the dose sleeve 30 and to the scale drum 35 by the overload clutch (collar 36). Due to the threaded connection with the clutch sleeve 20, the dose sleeve 30, the collar 36, the scale drum 35 and the dose knob 50 are moved in proximal direction.

As the drive sleeve 40 is rotationally coupled to the dose sleeve 30 the drive sleeve 40 is rotated too together with the dosing click ring 80. Therefore, the dosing click teeth 82 slide over the counter dosing click teeth 72 of the ratchet sleeve 70 (which is not moved) thereby generating a click sound.

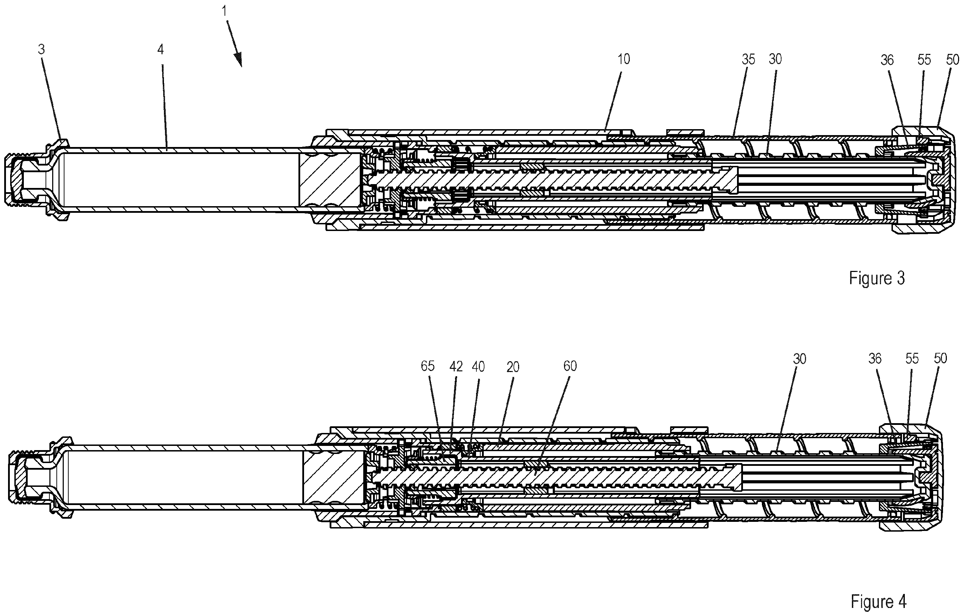

Figure 3 depicts a further sectional view of the injection pen 1 when the dose sleeve 30 is in a proximal end position when a maximum dose is set. During dose setting the drive member 40 is in its proximal dose setting position and hence not coupled to the drive nut 65 and thus the drive nut 65, the dispensing click disc 90 and the plunger rod 60 are not rotated relative to the housing 10. The clutch sleeve 20 cannot rotate as it is splined to the housing 10. As the drive sleeve 40 is rotated relative to the plunger rod the stop nut 67 is screwed in proximal direction during dose setting.

To correct a dose the user can rotate the dose knob 50 in counter direction to decrease the dose and thus the dose sleeve 30 is screwed back into the housing.

Once the correct dose is set the user presses onto dose knob 50 and shifts the dose knob 50 relative to the collar 36 in distal direction. This state is depicted in figure 4. The axial travel of the dose knob 50 moves the grooves 55 out of engagement with the cams 37 and thus rotationally decouples the dose sleeve 30 from the dose knob 50. Furthermore,

The axial user force acts on the dose sleeve 30 via bearing 51 and the dose sleeve pushes the clutch sleeve 20 via threaded engagement distally. As the connecting disc 45 abuts on a ledge of the drive sleeve 40, the drive sleeve 40 is moved from its dose setting position distally into its dispensing position.

That means the drive sleeve 40 is moved distally relative to the dosing click ring 80 and thus the drive sleeve protrusions get out of engagement with the ring grooves and thus the drive sleeve rotationally decouples from the dosing click ring 80. The drive sleeve 40 is also moved relative to the drive nut 65 and the coupling portion 42 with the splines 41 (protrusions or grooves) are moved onto the splines 66 (protrusions or grooves) on an outer proximal coupling portion of the drive nut 65. As shown in figure 4 the drive sleeve 40 is thus rotationally coupled to the drive nut 65 and thus to the plunger rod 60.

When the user further presses the dose knob 50 in distal direction the dose sleeve 30 is screwed back into the housing 10. The drive sleeve 40 is rotated in dispensing direction and the rotationally coupled drive nut 65 is rotated too which in turn is rotationally coupled to the plunger rod 60 and thus transfers the rotation to the plunger rod 60. As the latter is in threaded connection with the bearing disc 18 the plunger rod 60 is screwed in distal direction. This moves the piston inside the cartridge barrel in distal direction to dispense the liquid drug.

The rotation of the drive nut 65 causes the dispensing click disc 90 to rotate as it is rotationally coupled to the drive nut. The dispensing click teeth 82 thus slide over the counter dispensing click teeth 72 of the clutch sleeve 70 (see figure 2 or figure 5 and 6) and thereby generate a click sound during dose dispensing. The saw teeth form of the dispensing click teeth 82 and counter dispensing click teeth 72 allow a rotation of the plunger rod in dispensing direction and prevent a rotation in counter direction.

During dose dispensing the drive sleeve 40 rotates together with the plunger rod 60 and hence there is no relative rotation therebetween. Therefore, the stop nut 67 does not move relative to the plunger rod 60 and remains in its position.

In order to prepare the injection pen 1 for a new injection and to reset the dispensing mechanism the user decouples the cartridge holder 2 from the housing 10 by a rotation and subsequently by a linear retraction movement to disconnect the bayonet connection between the cartridge holder 2 and the housing 10.

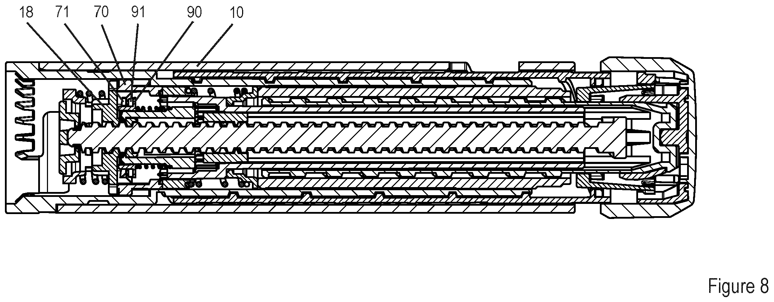

When a proximal end rim of the cartridge holder 2 is moved distally away from the cartridge support 6 the latter is moved distally by the biased cartridge spring 7. As the cartridge holder no longer presses onto the u-shaped rails 74 the rachet sleeve 70 is moved distally relative to the housing, see figure 8. The linear displacement is guided in the linear guide 11 (figure 2). The dose and dispensing mechanism is then in a reset configuration as shown in figure 8. That means the reverse lock provided by the saw teeth form of the dispensing click teeth 91 of the dispensing click disc 90 and the saw teeth form of the counter dispensing click teeth 72 is released and the plunger rod 60 can be pushed back into the housing 10 by the user. To that end, the user presses on the distal surface of the non-rotating flange 67 and forces the latter proximally, leading to reverse rotation of plunger rod and drive nut. When a new cartridge 3 is inserted into cartridge holder 2 the user can reattach it to the housing 10 via bayonet connection. When the proximal end of the cartridge holder 2 abuts the cartridge support 6 the ratchet sleeve 70 is moved proximally and thereby the dispensing click teeth 91 engage the counter dispensing click teeth 72 and thus the reverse lock is active again. A subsequent rotation of the cartridge holder 2 relative to the housing 10 closes the bayonet connection.

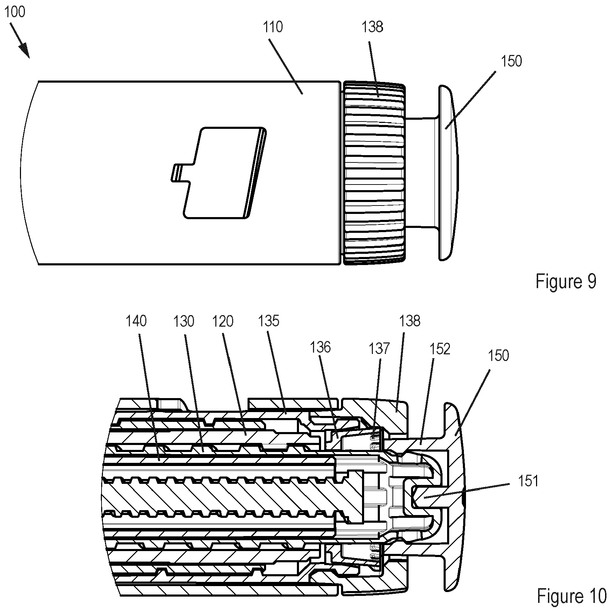

Figure 8 depicts a proximal end of a further embodiment of an injection pen 100 and figure 9 depicts a sectional view of the proximal end of this injection pen.

In figure 9 a dose setting position of the clutch sleeve 120, the dose sleeve 130 and a dispensing button 150 is shown. In contrast to the embodiment shown in figures 1 to 7 in the embodiment according to figure 8 and 9 the injection pen 100 comprises a two-part dose setting knob including a dose setting ring 138 and the axially movable dispensing button 150.

The user rotates the dose setting ring 138 in order to set a dose. If a desired dose is set the user presses the dispensing button 150 in distal direction. That means a non-rotating cylindrical portion 152 of the button 150 presses on a proximal end of the collar 137 of the dose sleeve 130 and thereby shifts the collar 137 relative to the dose setting ring 138 (and relative to the housing 110). That in turn means the cams of the collar move out of grooves in the dose setting ring 138 and thus the collar 137 with the dose sleeve 130 are rotationally decoupled from the dose set ring. The collar 137 provides an overload clutch as described above with respect to the first embodiment.

When the user further presses the button in distal direction the dose sleeve 130, the scale drum 135 and the clutch sleeve 120 are moved distally. The dose sleeve 130 is screwed back into the housing and the rotating drive sleeve 140 drives the plunger rod in dispensing direction as described with respect to the first embodiment.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | |
|---|---|---|---|
| 1 | injection pen | 70 | ratchet sleeve |
| 2 | cartridge holder | 71 | counter dispensing click teeth |
| 3 | cartridge | 72 | counter dosing click teeth |
| 4 | needle | 73 | protrusion |
| 5 | cap | 74 | guiding rails |
| 6 | cartridge support | 75 | guiding protrusion |
| 7 | cartridge spring | | |
| 10 | housing | 80 | dosing click ring |
| 11 | linear guide | 82 | dosing click teeth |
| 15 | insert sleeve | | |
| 18 | bearing disc | 85 | reset spring |
| 20 | clutch sleeve | 90 | dispensing click disc |
| 30 | dose sleeve | 91 | dispensing click teeth |
| 35 | scale drum | 92 | spring |
| 36 | coupling collar | 93 | cams |
| 37 | cams | | |
| 40 | drive sleeve | 100 | injection pen |
| 41 | splines | 110 | housing |
| 42 | coupling part | 120 | clutch sleeve |
| 45 | connecting disc | 130 | dose sleeve |
| 50 | dose knob | 136 | coupling collar |
| 51 | bearing | 137 | cams |
| 55 | knob coupling portion | 138 | dose set ring |
| 60 | plunger rod | 140 | drive sleeve |
| 61 | outer thread | 150 | dispensing button |
| 62 | end thread segment | 151 | bearing |
| 63 | counter stop surface | 152 | cylindrical portion |
| 64 | flange | | |
| 65 | drive nut | | |
| 66 | splines | | |
| 67 | stop nut | | |
| 68 | stop surface | | |
| 69 | cam | | |

## Claims

1. A manually driven and reusable injection device (1) for dispensing a liquid drug from a reservoir (3) through an injection needle (4), the device comprising
- a housing (10) defining longitudinal axis;
- a dose setting member (30) inside the housing and movable out of the housing for setting a dose, wherein an axial position of the dose setting member (30) relative to the housing is indicative of a set dose;
- a plunger rod (60) displaceable relative to the housing for dispensing the liquid drug from the reservoir (3);
- a dosing click member (80) inside the housing, comprising axially extending dosing click teeth (82) adapted to engage axially extending counter dosing click teeth (72);
- a dispensing click member (90) inside the housing, comprising dispensing click teeth (91) adapted to engage counter dispensing click teeth (71);
- a drive member (40) operatively coupled to the dose setting member, wherein the drive member can be in a dose setting position or in a dispensing position,
wherein in the dose setting position the drive member (40) is operatively coupled to the dosing click member (80) to rotate the dosing click member (80) relative to the housing which causes the dosing click teeth (82) to slide over the counter dosing click teeth (72);
wherein in the dispensing position the drive member (40) is decoupled from the dosing click member (80) and operatively coupled to the dispensing click member (90) to rotate the dispensing click member (90) relative to the housing which causes the dispensing click teeth (91) to slide over the counter dispensing click teeth (71) and wherein the drive member (40) is further operatively coupled to the plunger rod (60),
**characterized by** a ratchet member (70) comprising the counter dosing click teeth (72) and the counter dispensing click teeth (71) which are distinct from the counter dosing click teeth and wherein the ratchet member (70) can be in first axial position in which the counter dispensing click teeth (71) engage the dispensing click teeth (91) and in a second axial position in which the counter dispensing click teeth (71) are axially separated from the dispensing click teeth (91) wherein the plunger rod (60) comprises a flange (64) rotatably connected to plunger rod with a distal surface adapted to serve as a contact for user-initiated force application for proximal displacement of the plunger rod (60) when the ratchet member (70) is in its second axial position and wherein the plunger rod is operatively connected to the housing (10) by a threaded connection.

2. Injection device according to claim 1, wherein the dosing click member (80) is sleeve shaped and the dosing click teeth (82) are arranged on a front surface of the sleeve-shaped dosing click member.

3. Injection device according to claim 2, wherein the drive member (40) is arranged coaxially inside the sleeve-shaped dosing click member (80).

4. Injection device according to any of claims 1 to 3, wherein the drive member (40) and the dosing click member (80) are axially movable relative to each other for rotationally coupling or decoupling.

5. Injection device according to any of claims 1 to 4, wherein in the dose setting position the drive member (40) is rotationally decoupled from the plunger rod (60) and in the dispensing position the drive member (40) is rotationally coupled to the plunger rod (60) and preferably also to the dispensing click member (90).

6. Injection device according to any of claims 1 to 5, wherein the counter dosing click teeth (72) and the counter dispensing click teeth (71) of the ratchet member (70) are both arranged in a respective circle, and wherein the circles are coaxially positioned.

7. Injection device according to any of claims 1 to 6, wherein the ratchet member (70) is sleeve shaped and wherein the dispensing click member (90) is arranged coaxially inside the sleeve-shaped ratchet member.

8. Injection device according to claim 7, wherein the counter dosing click teeth (72) are arranged on an annular front surface of the sleeve-shaped ratchet member (70) and wherein the counter dispensing click teeth (71) are arranged on an annular inner surface perpendicular to the longitudinal axis.

9. Injection device according to any of claims 1 to 8, wherein the ratchet member (70), the dosing click member (80), and the dispensing click member (90) are concentrically arranged to each other.

10. Dose and dispensing mechanism according to any of claims 1 to 9, comprising a stop member (67) in threaded connection with the plunger rod (60) and splined to the drive member (40) such that the stop member (67) is movable axially relative to the drive member (40) but is prevented from being rotated relative to the drive member (40).

11. Injection device according to any of claims 1 to 10, comprising a drive nut (65) which is rotationally coupled to the plunger rod (60) but is movable relative to the plunger rod and the drive nut is coaxially inside drive member (40), wherein the drive nut (65) can be releasably and rotationally coupled to drive member (40).

12. Injection device mechanism according to claim 11, wherein the drive member (40) comprises a sleeve-shaped coupling portion including a protrusion or groove and the drive nut (65) includes the other of the protrusion and groove to releasably and rotationally couple the drive nut (65) to the drive member (40).

13. Injection device mechanism according to claim 11 or 12, wherein the dispensing click member (90) is rotationally coupled to the plunger rod (60) by the drive nut (65).

14. Injection device according to any of claims 1 to 13, further comprising a clutch member (20) axially movable relative to the housing (10) and rotationally coupled to the housing (10) and in threaded connection with a dose member (30) and wherein an axial movement of the dose member (30) is transferred by the clutch member to the drive member (40) to move the drive member between the dose setting position and dispensing position.

15. Injection device according to claim 14, wherein the clutch member (20) comprises an inner thread and dose member (30) comprises an outer thread engaging the inner thread.
